# EUROPEAN PATENT APPLICATION

(11) **EP 4 026 523 A1**
(43) Date of publication of application: **13.07.2022**
(21) Application number: 21217160.7
(22) Date of filing: 22.12.2021
(51) Int. Cl.: A61F 2/70

(54) **POWER CONSUMPTION CALCULATION SYSTEM FOR PROSTHETIC LEG, STATE ESTIMATION SYSTEM FOR PROSTHETIC LIMB, AND PHYSICAL CONDITION ESTIMATION SYSTEM FOR USER OF PROSTHETIC LIMB**

(30) Priority: 22.12.2020 JP 2020212849
(71) Applicant: Nabtesco Corporation, Tokyo 102-0093 (JP)
(72) Inventor: Oka, Daisuke, Tokyo (JP); Wang, Zhiqin, Tokyo (JP); Toyoda, Takashi, Tokyo (JP)
(74) Representative: Grünecker Patent- und Rechtsanwälte PartG mbB

(57) **Abstract**

A prosthetic leg system includes a prosthetic leg (10), a system controller (100), and a system memory (200). The system controller includes a driving information acquirer (101) structured to acquire driving information representing a driving state of a driving mechanism (40) of the prosthetic leg and an environmental information acquirer (102) structured to acquire environmental information around the prosthetic leg. The system controller configures a system for calculating power consumption of the prosthetic leg, a system for estimating a state of the prosthetic leg, and a system for estimating a physical condition of a user of the prosthetic leg (Figure 5).

## Description

The present invention relates to a power consumption calculation system for a prosthetic leg, a state estimation system for a prosthetic limb, and a physical condition estimation system for a user of a prosthetic limb.

As a prosthetic limb, such as a prosthetic arm or a prosthetic leg, worn by a person who has lost a limb due to injury or illness, there is known a prosthetic limb capable of controlling bending and stretching of a joint provided at a joint portion such as an elbow, a knee, a wrist, or an ankle. Hydraulic pressure, pneumatic pressure, a spring, and the like are used for a driving mechanism of these joints.

Patent Literature 1: JP 2017 -6339 A

The present inventor has conducted unique studies from the viewpoint of further improving convenience of a prosthetic limb, and has recognized the following plurality of problems. A first problem is that, when the user of the prosthetic leg goes to a destination, power consumption of a battery increases depending on a route, and there is a risk that the amount of remaining power of the battery becomes insufficient on the way. The second problem is that even if there is no major problem in the prosthetic limb itself, abnormality may occur depending on the environment in which the prosthetic limb is used. The third problem is that even if there is no major problem in the prosthetic limb itself, abnormality may occur in the physical condition of the user.

The present invention has been made in view of such a situation, and an object thereof is to provide a highly convenient system related to a prosthetic limb.

In order to solve the above first problem, a power consumption calculation system for a prosthetic leg according to an aspect of the present invention includes: a route information acquirer structured to acquire route information indicating a route from a start point to an end point where a user wearing the prosthetic leg, including a driving mechanism for driving a knee joint with electric power supplied from a battery, can walk; a road surface shape information acquirer structured to acquire road surface shape information representing a shape of a road surface of the route; a power consumption calculator structured to calculate, based on the road surface shape information, power consumption of the battery at a time when the user wearing the prosthetic leg walks on the route, ; and an output unit structured to output the calculated power consumption.

According to this aspect, since the power consumption calculated based on the road surface shape can be recognized when the user of the prosthetic leg goes to the destination, it is possible to take appropriate measures such as avoiding a route with large power consumption and charging the battery.

In order to solve the above second problem, a state estimation system according to an aspect of the present invention is a state estimation system for a prosthetic limb including a driving mechanism that drives a joint, the system including: a driving information acquirer structured to acquire driving information representing a driving state at present of the driving mechanism; an environmental information acquirer structured to acquire environmental information at present around the prosthetic limb; an estimator structured to estimate a state of the prosthetic limb based on the driving information and the environmental information; and an output unit structured to output a result of the estimation.

According to this aspect, even if there is no major problem in the prosthetic limb itself, it is possible to recognize abnormality that can occur according to the environment in which the prosthetic limb is used.

In order to solve the third problem, a physical condition estimation system according to an aspect of the present invention is a physical condition estimation system for a user of a prosthetic limb including a driving mechanism that drives a joint, the system including: a driving information acquirer structured to acquire driving information representing a driving state of the driving mechanism; a reference information acquirer structured to acquire reference information in which the driving information and the physical condition information of the user are associated with each other when the user wore the prosthetic limb in the past; and an estimator structured to estimate a physical condition of the user at present, based on comparison of the driving information at present while the user wears the prosthetic limb and the reference information.

According to this aspect, even if there is no major problem in the prosthetic limb itself, it is possible to recognize the abnormality in the physical condition of the user who uses the prosthetic limb.

The systems of the above aspects have different problems to be solved, but are common in that convenience is enhanced by processing of various types of information acquired from the inside and outside of the prosthetic limb.

Note that any combinations of the above components and modifications of the expressions of the present invention for conversion between the methods, apparatuses, systems, recording media, computer programs, and the like of the invention are also effective as aspects of the present invention.

According to the present invention, it is possible to provide a highly convenient system related to a prosthetic limb.
FIG. 1 is a diagram illustrating a schematic configuration of a prosthetic leg to which various systems according to an embodiment are applied;
FIG. 2 is a diagram illustrating a schematic configuration of the prosthetic leg to which various systems according to the embodiment are applied;
FIG. 3 is a diagram illustrating a cylinder and a driving mechanism of the prosthetic leg;
FIGS. 4A and 4B are diagrams illustrating flows of oil at times of bending and extending a knee unit of the prosthetic leg;
FIG. 5 is a functional block diagram schematically illustrating configurations of various prosthetic leg systems;
FIG. 6 is a view illustrating an example of calling attention to a user of the prosthetic leg; and
FIG. 7 is a view illustrating another example of calling attention to the user of the prosthetic leg.

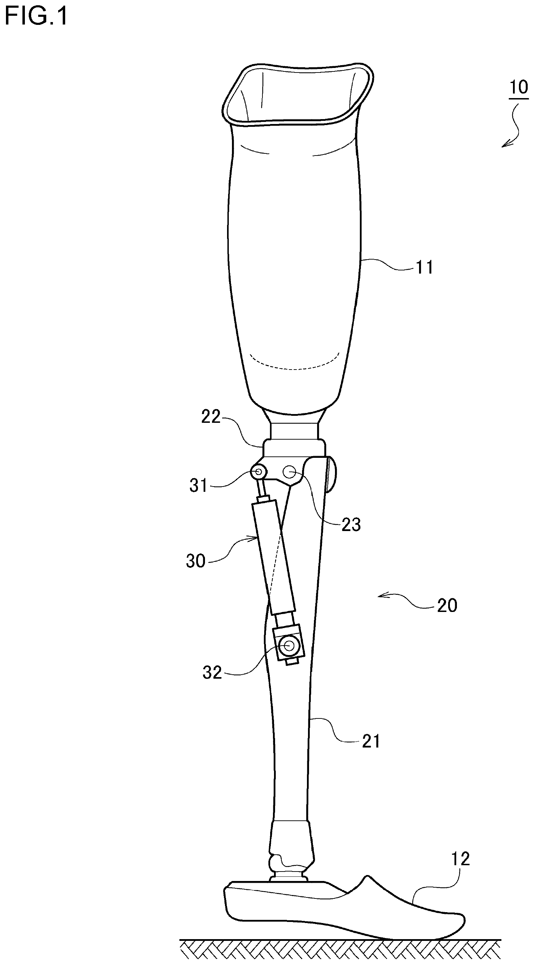

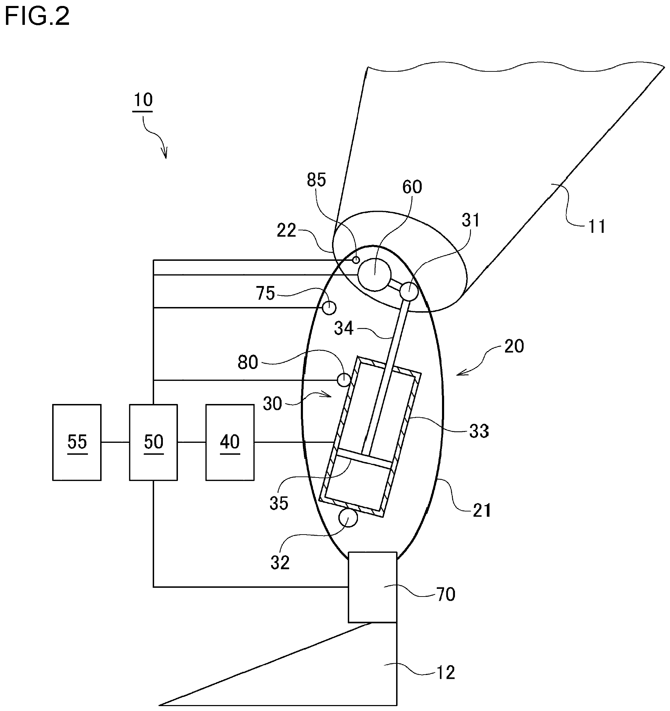

FIGS. 1 and 2 illustrate a schematic configuration of a prosthetic leg 10, in which a knee joint performing a knee function can be driven, as an example of a prosthetic limb to which various systems according to an embodiment of the present invention are applied.

The prosthetic leg 10 includes a plastic socket 11 corresponding to the thigh, a knee joint 20 corresponding to the lower leg coupled to the lower end of the socket 11, and a foot 12 coupled to the lower end of the knee joint 20. The socket 11 and the knee joint 20 are rotatably provided around a rotation axis perpendicular to the paper surface of FIG. 1 through a support point 23 provided at a coupling portion thereof, and enable bending and stretching of the knee unit 22. In addition, the knee joint 20 and the foot 12 are coupled by an elastic member, and the relative posture of the knee joint 20 and the foot 12 is kept constant when the load from the ground contact surface is small, such as when the artificial leg 10 is not grounded or is upright. In addition, when a load from the ground contact surface is large during walking or the like, the elastic member is elastically deformed to generate a propulsive force for kicking out the ground contact surface.

The knee joint 20 includes a high-strength frame 21 having a shape resembling a lower leg, a knee unit 22 fixed to the socket 11 and coupled to the frame 21 turnably about the rotation axis, a cylinder 30 that restricts or assists the rotational operation of the knee unit 22, and a driving mechanism 40 for driving the cylinder 30.

The amount of expansion and contraction of the cylinder 30 and the rotation angle (bending and stretching angle) of the knee unit 22 correspond one-to-one, and a knee angle sensor 60 that measures the amount of expansion and contraction of the cylinder 30 and detects the angle of the knee unit 22 is provided near the cylinder 30 and the knee unit 22. The knee angle detected by the knee angle sensor 60 is used by the controller 50. The knee angle is an angle formed by the axis of the socket 11 and the axis of the frame 21. For example, as illustrated in FIG. 1, when a user of the prosthetic leg 10 is upright and the axis of the socket 11 and the axis of the frame 21 are on a straight line, the knee angle is 0 °. In addition, in a case where the user of the prosthetic leg 10 sits down, the axis of the frame 21 remains in the vertical direction in FIG. 1, and the axis of the socket 11 changes in the horizontal direction, the knee angle becomes 90 °.

A load sensor 70 that detects a load (vertical load) of the knee joint 20 with respect to the foot 12 is provided at the lower end of the frame 21. In addition to or instead of the load sensor 70, an acceleration sensor 75 is provided near the knee unit 22 in the frame 21. Furthermore, a temperature sensor 80 is attached to the cylinder 30. The measurement information of each of these sensors is used by the controller 50.

The knee unit 22 is provided with a vibrator 85. The vibrator 85 notifies or calls attention to the user wearing the prosthetic leg 10 by vibration, and is controlled by the controller 50.

The controller 50 controls the driving mechanism 40 based on measurement information of various sensors such as the knee angle sensor 60, the load sensor 70, the acceleration sensor 75, and the temperature sensor 80 and drives the cylinder 30 to expand and contract. A battery 55 that supplies power to each unit of the prosthetic leg 10 is connected to the controller 50. Note that, in FIG. 2, the driving mechanism 40, the controller 50, and the battery 55 are illustrated outside the knee joint 20, but are actually provided on the inside of the frame 21 or integrally with the cylinder 30 as components of the knee joint 20.

The cylinder 30 is a hydraulic cylinder that uses oil as a working fluid and generates drag to limit or assist the operation of the knee unit 22. The cylinder 30 is supported by an upper support point 31 provided near a support point 23 turnably coupling the socket 11 and the frame 21 and a lower support point 32 coupled to a part of the frame 21, and is extendable and contractible between both support points. In a reduction step in which a cylinder length decreases, the knee unit 22 performs the bending operation to rotate in the counterclockwise direction in FIG. 1, and in an extension step in which the cylinder length increases, the knee unit 22 performs the extension operation to rotate in the clockwise direction in FIG. 1. Here, the cylinder length refers to a length between the upper support point 31 and the lower support point 32 of the cylinder 30.

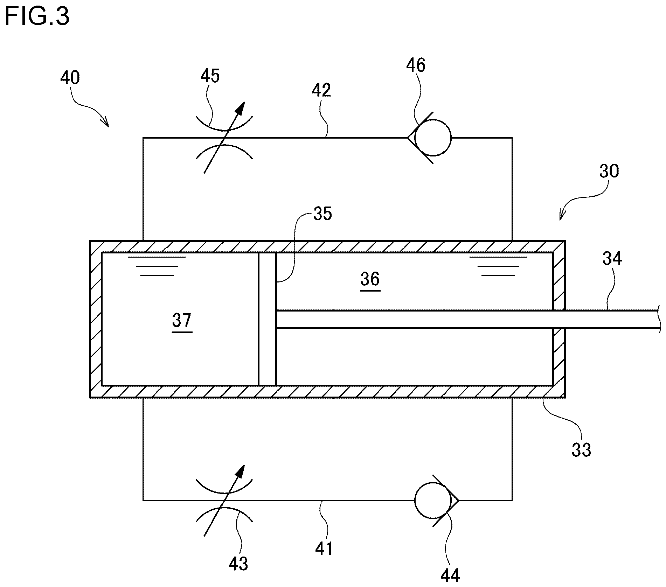

Next, the cylinder 30 and the driving mechanism 40 are explained with reference to FIG. 3. The cylinder 30 includes a cylinder tube 33, a piston rod 34 inserted from one end side (right end side in FIG. 3) of the cylinder tube 33 and movable along a longitudinal direction of the cylinder tube 33 (left-right direction in FIG. 3), and a piston 35 fixed to the piston rod 34 in the cylinder tube 33 and slidable in the longitudinal direction along an inner wall of the cylinder tube 33. The inside of the cylinder tube 33 is divided by the piston 35 into a first cavity 36 on one end side (right end side in FIG. 3) and a second cavity 37 on the other end side (left end side in FIG. 3). The first cavity 36 and the second cavity 37 are filled with oil that is a working fluid.

The driving mechanism 40 is a hydraulic driving mechanism that drives the cylinder 30 to expand and contract with hydraulic pressure. The driving mechanism 40 includes an extension-side hydraulic circuit 41 and a bending-side hydraulic circuit 42 each connected to the cylinder 30. The extension-side hydraulic circuit 41 and the bending-side hydraulic circuit 42 communicate with the first cavity 36 on one end side and communicate with the second cavity 37 on the other end side, respectively. The extension-side hydraulic circuit 41 includes an extension-side valve 43 functioning as a hydraulic valve and an extension-side check valve 44. When the extension-side valve 43 is opened, oil can flow through the extension-side hydraulic circuit 41, but because of the action of the extension-side check valve 44, the oil flows only in the direction from the first cavity 36 toward the second cavity 37 and does not flow in the opposite direction. The bending-side hydraulic circuit 42 includes a bending-side valve 45 functioning as a hydraulic valve and a bending-side check valve 46. When the bending-side valve 45 is opened, the oil can flow through the bending-side hydraulic circuit 42, but because of the action of the bending-side check valve 46, the oil flows only in the direction from the second cavity 37 toward the first cavity 36 and does not flow in the opposite direction. The opening degrees of the extension-side valve 43 and the bending-side valve 45 are individually controlled by the controller 50. The opening degree of each valve can take any value between the full opening (maximum opening degree) and the full closing (minimum opening degree) thereof. When each valve is fully closed, the flow of oil is blocked, and the hydraulic resistance is maximized. In addition, as the opening degree of each valve increases toward the full opening, the cross-sectional area through which the oil can flow in each valve increases, so that the flow rate of the oil increases and the hydraulic resistance decreases.

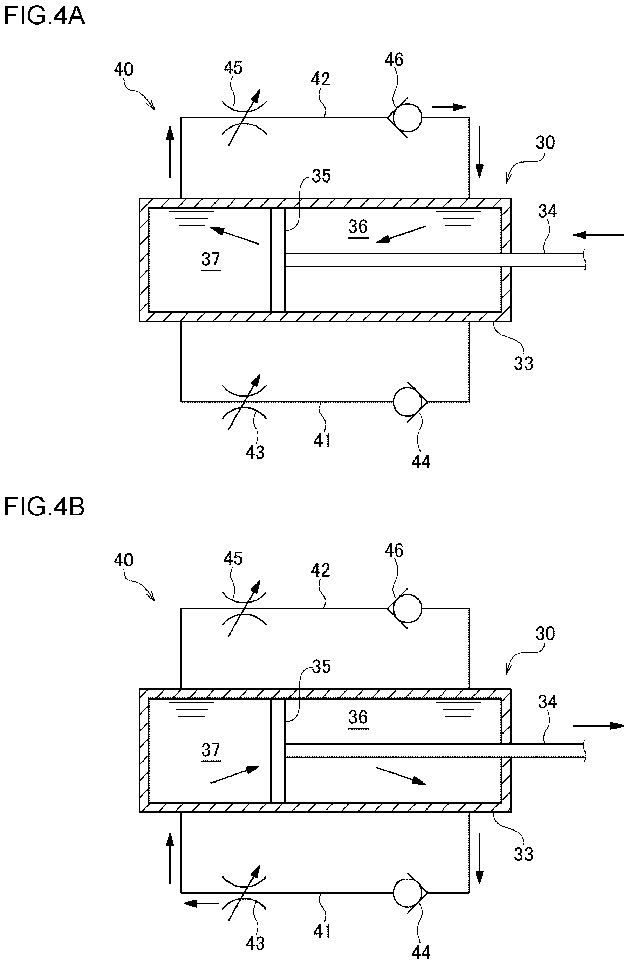

FIG. 4A illustrates the flow of oil at a time of bending the knee unit 22. The bending is a reduction step in which the cylinder length decreases. The piston rod 34 is retracted to the left in FIG. 4 and the piston 35 moves to the retracting side. Since the oil pushed out of the second cavity 37 by the movement of the piston 35 cannot flow through the extension-side hydraulic circuit 41 including the extension-side check valve 44, the oil flows through the bending-side hydraulic circuit 42 and flows into the first cavity 36. At this time, if the opening degree of the bending-side valve 45 is lowered, the oil can be prevented from easily flowing through the bending-side hydraulic circuit 42, so that the bending operation of the knee unit 22 can be restricted.

FIG. 4B illustrates the flow of oil at a time of extending the knee unit 22. The extension is an extension step in which the cylinder length increases. The piston rod 34 extends to the right in FIGS. 4A and 4B and the piston 35 moves to the extrusion side. Since the oil pushed out of the first cavity 36 by the movement of the piston 35 cannot flow through the bending-side hydraulic circuit 42 including the bending-side check valve 46, the oil flows through the extension-side hydraulic circuit 41 and flows into the second cavity 37. At this time, if the opening degree of the extension-side valve 43 is lowered, the oil can be made difficult to flow through the extension-side hydraulic circuit 41, so that the extension operation of the knee unit 22 can be restricted.

Returning to FIG. 2, various sensors provided in the prosthetic leg 10 are explained.

The knee angle sensor 60 measures a telescopic position of the piston rod 34. For example, the position of a magnet attached to the piston rod 34 is measured by a magnetic sensor provided in the cylinder tube 33. Since the expansion/contraction position of the piston rod 34 and the bending/stretching angle of the knee unit 22 correspond one-to-one, the knee angle sensor 60 can convert the measured expansion/contraction position of the piston rod 34 into the angle of the knee unit 22. Note that the calculation for converting the expansion/contraction position of the piston rod 34 into the angle of the knee unit 22 may be performed by the controller 50. In this case, the knee angle sensor 60 measures the expansion/contraction position of the piston rod 34 and provides the expansion/contraction position to the controller 50.

The load sensor 70 includes, for example, a strain sensor and is provided at the ankle between the frame 21 and the foot 12. Since the load applied to the ankle causes strain in an object configuring the strain sensor, the load can be measured by detecting the strain. Such a load sensor may be provided in the knee unit 22 or another joint unit to measure the load of each joint unit. In a prosthetic arm as another example of a prosthetic limb, it is preferable to provide a load sensor in a joint portion such as a wrist portion, an elbow portion, and a shoulder portion. Note that the calculation for converting the strain detected by the strain sensor into a load may be performed by the controller 50. In this case, the load sensor 70 provides the detected strain to the controller 50. In addition, the controller 50 can obtain a stride and walking speed by calculation based on the loads at the time of leaving and landing for each step and the duration of a small load obtained from the load sensor 70. Alternatively, the stride and the walking speed may be estimated from measurement information of the load sensor 70 based on machine learning data indicating a correlation between the transition of the load during walking and the stride and the walking speed.

The movement of the prosthetic leg 10 can be detected based on each piece of information of acceleration measured by the acceleration sensor 75, speed obtained by integrating the acceleration, and a position obtained by integrating the speed. The controller 50 performs calculation to obtain the speed and the position from the acceleration. At that time, since the controller 50 can detect the departure and landing for each step from an acceleration change, it is possible to obtain a change in position per step, that is, the stride.

The temperature sensor 80 measures the temperature of oil that is the working fluid of the cylinder 30. Since a hydraulic resistance changes according to a temperature change because of the physical properties of the oil, the controller 50 controls the driving mechanism 40 to realize a desired hydraulic resistance corresponding to the temperature measured by the temperature sensor 80. Specifically, the controller 50 stores, for each temperature, a control data set to the driving mechanism 40 for realizing each value of the hydraulic resistance. The controller 50 selects a control data set corresponding to the temperature measured by the temperature sensor 80 and uses the control data set for control. Note that the temperature sensor 80 may be provided in another place of the prosthetic leg 10. For example, by providing the temperature sensor 80 on the surface of the frame 21, the air temperature around the prosthetic leg 10 can be measured. In addition, the temperature sensor 80 may be provided near a control board of the controller 50 that controls the driving mechanism 40.

The controller 50 is a central processing unit that controls the entire prosthetic leg 10. The controller 50 controls the driving mechanism 40 based on measurement information of various sensors such as the knee angle sensor 60, the load sensor 70, the acceleration sensor 75, and the temperature sensor 80 and drives the cylinder 30 to expand and contract. Although the controller 50 can be caused to execute any control program, several specific control examples are explained below.

### (1) Control of a lock angle of the prosthetic leg

A lock angle of the prosthetic leg 10 is set according to the user of the prosthetic leg 10 and a use situation. The lock angle is a maximum bendable angle of the knee unit 22 during normal walking. For example, a user whose muscle strength and walking ability are deteriorated due to old age, illness, injury, or the like may lose his/her body balance and fall down when the bending angle of the knee unit 22 increases. Therefore, safety is improved by setting the lock angle small. In addition, since there is danger of falling down when walking in a place with a gradient such as a staircase or a slope, the lock angle is reduced to encourage low-speed walking at a small stride. In such control, the controller 50 constantly monitors the angle of the knee unit 22 measured by the knee angle sensor 60, and when the angle of the knee unit 22 is equal to or larger than the lock angle, the bending-side valve 45 is fully closed, so that the hydraulic resistance on the bending side is maximized and the knee unit 22 is limited not to be bent any more. In addition, in a case where the angle of the knee unit 22 is less than the lock angle, the opening degree of the bending-side valve 45 is lowered as the angle approaches the lock angle, so that it is possible to surely perform control so that the angle does not exceed the lock angle. In addition, when walking in a place with a gradient such as a staircase or a slope, the load measured by the load sensor 70 increases. Therefore, in a case where the load measured by the load sensor 70 exceeds a predetermined threshold, the controller 50 determines that the user walks in a place with a gradient, and can reduce the lock angle. Note that the detection of the gradient may be performed by the acceleration sensor 75.

### (2) Control based on temperature

As temperature rises, the hydraulic resistance of the cylinder 30 decreases due to the physical properties of the oil. Therefore, in order to obtain a desired hydraulic resistance, it is necessary to set the opening degree of each of the hydraulic valves 43 and 45 to be lower than that in the normal time. In such control, the controller 50 constantly monitors the temperature of the cylinder 30 measured by the temperature sensor 80 and adjusts the opening degree of each of the hydraulic valves 43 and 45 according to the temperature.

### (3) Control based on acceleration

The acceleration sensor 75 can detect the magnitude and direction of a motion of the user wearing the prosthetic leg 10. From these pieces of information, the controller 50 can calculate basic information of walking such as a stride and walking speed and information regarding a walking place such as the magnitude of a gradient. The controller 50 can adjust the control of the driving mechanism 40 according to the calculated stride and walking speed. For example, when the stride and the walking speed are small, the bending angle of the knee unit 22 may be small, and thus the lock angle is set to be small. In addition, in a case where the stride and the walking speed are small, the balance of the body at the time of walking is less likely to be lost. Therefore, by increasing the opening degree of each of the hydraulic valves 43 and 45, the hydraulic resistance may be lowered to make it easy to bend and stretch the knee unit 22. On the other hand, when the stride and the walking speed are large, the lock angle is set to be large so that the knee unit 22 can be largely bent, and the opening degree of each of the hydraulic valves 43 and 45 is lowered to increase the hydraulic resistance, and sudden bending and stretching of the knee unit 22 is prevented to maintain the balance of the body during walking. Similarly, when walking in a place with a large gradient, it is preferable to set the lock angle to be large and increase the hydraulic resistance.

### (4) Safety lock

Safety lock is to restrict further bending for safety when the knee unit 22 remains still while being bent. In this case, the controller 50 calculates angular velocity of the knee unit 22 by differentiating the angle of the knee unit 22 measured by the knee angle sensor 60. In a case where a state in which the angle of the bent knee unit 22 is equal to or greater than a predetermined value and the angular velocity of the knee unit 22 is less than a predetermined value continues for a certain period of time, the controller 50 determines that the knee unit 22 is in a stationary state. Then, it is considered that the user of the prosthetic leg 10 intends to maintain the stationary state, and further bending is restricted for safety. Specifically, by bringing the bending-side valve 45 into a fully closed state, the hydraulic resistance on the bending side is maximized, and the safety lock is applied so that the knee unit 22 is not bent any more. In a state in which the safety lock is applied, the stationary state is maintained without power of the user, so that the user can maintain a half-sitting posture of the user without getting tired. Note that the safety lock is released when the user extends the knee unit 22 and oil at a predetermined flow rate or more flows through the extension-side hydraulic circuit 41.

The prosthetic leg 10 as an example of the prosthetic limb is explained above. Next, various systems for improving the convenience of such a prosthetic leg 10 are explained.

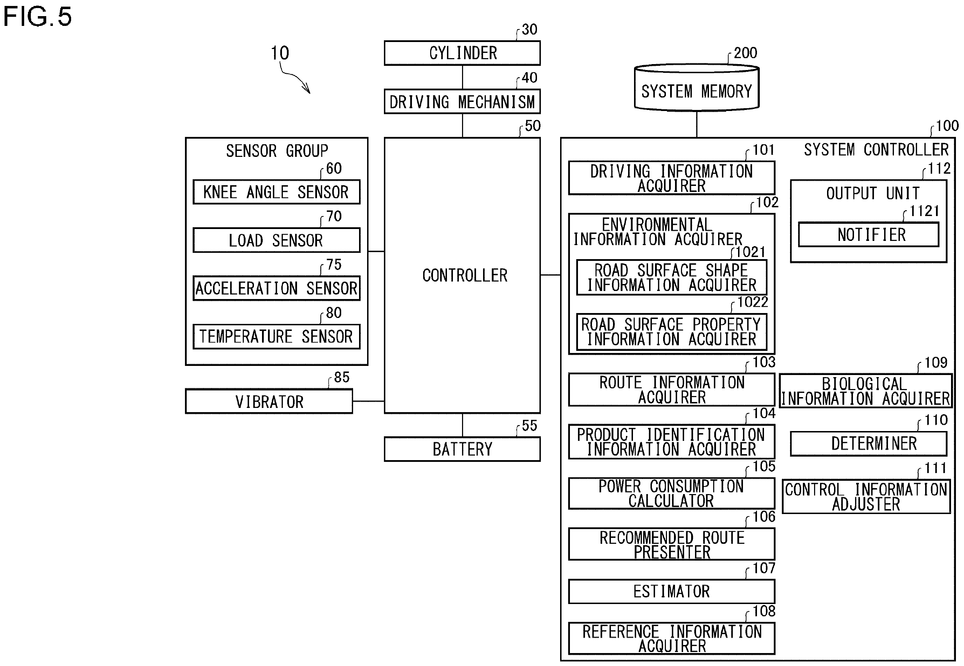

FIG. 5 is a functional block diagram schematically illustrating a configuration of such various prosthetic leg systems. Each functional block illustrated in this drawing can be selected according to a system to be configured, and it is not always necessary to provide all functional blocks.

The prosthetic leg system includes the prosthetic leg 10, a system controller 100, and a system memory 200. The system controller 100 is realized by an information processing apparatus outside the prosthetic leg 10 and performs control of the entire prosthetic leg system and information processing. Here, examples of the information processing apparatus include general-purpose electronic devices such as a smartphone, a tablet, and a computer. These electronic devices may be used by the user of the prosthetic leg 10 or may be used by an outsider other than the user. The system controller 100 is capable of wirelessly communicating with the controller 50 of the prosthetic leg 10 based on a short-range wireless communication standard such as Bluetooth (registered trademark), a mobile communication standard such as LTE or 5G, a wireless LAN standard such as Wi-Fi, or the like. In addition, the system controller 100 can communicate with any other communication devices via an information communication network such as the Internet. The system memory 200 stores various kinds of information used by the system controller 100. The system memory 200 may be provided in the same electronic device as an electronic device in which the system controller 100 is provided or may be provided in a remote server or the like accessible by the system controller 100 via the information communication network.

The system controller 100 includes a driving information acquirer 101, an environmental information acquirer 102, a route information acquirer 103, a product identification information acquirer 104, a power consumption calculator 105, a recommended route presenter 106, an estimator 107, a reference information acquirer 108, a biological information acquirer 109, a determiner 110, a control information adjuster 111, and an output unit 112. The environmental information acquirer 102 includes a road surface shape information acquirer 1021 and a road surface property information acquirer 1022. The output unit 112 includes a notifier 1121.

Among the above components, the driving information acquirer 101 and the environmental information acquirer 102 commonly used in various prosthetic leg systems explained later are explained.

The driving information acquirer 101 acquires driving information representing the driving state at present of the driving mechanism 40 of the prosthetic leg 10 from the controller 50 by wireless communication. The driving information may be any information as long as the information relates to the driving of the prosthetic leg 10, and is, for example, the following information.
- Measurement information of the knee angle sensor 60, the load sensor 70, the acceleration sensor 75, and the temperature sensor 80 included in a sensor group
- Internal state information representing an internal state of the cylinder 30 or the driving mechanism 40
- Walking information representing a walking state of the user calculated by the controller 50 based on the measurement information or the internal state information
- Remaining power information of the battery 55

The internal state information is, for example, the following information.
- Hydraulic resistance of each part of cylinder 30 or driving mechanism 40
- Position, speed, and acceleration of the piston 35
- Flow rate of oil flowing through hydraulic circuits 41 and 42
- Opening degree, opening/closing speed, opening/closing acceleration of each of hydraulic valves 43 and 45
- Convergence time until the opening degree of each of the hydraulic valves 43 and 45 converges to a command value from the controller 50
- Cumulative number of times of opening/closing and opening/closing time of each of the hydraulic valves 43 and 45

The walking information is, for example, the following information.
- Stride
- Walking speed
- Number of steps
- Number of times of bending and stretching and bending and stretching time of knee unit 22
- Kicking force for each step and impact at the time of landing (can be calculated from measurement information of acceleration sensor 75)
- Gradient of a walking place

Each of the kinds of information described above is calculated by the controller 50, but a part or all of the calculation may be performed by the driving information acquirer 101 instead.

Each of the pieces of driving information illustrated and enumerated above is acquired in real time from the prosthetic leg 10 worn by the user. The driving information acquirer 101 stores a part or all of the driving information acquired in real time in the system memory 200 as history driving information.

The environmental information acquirer 102 acquires environmental information at present around the prosthetic leg 10. Hereinafter, environmental information is illustrated and enumerated, and these pieces of information are acquired by a general-purpose electronic device implemented by the system controller 100 via the information communication network such as the Internet. Furthermore, in a case where the general-purpose electronic device in which the system controller 100 is realized is a smartphone, a wearable device, or the like carried by the user of the prosthetic leg 10, a part of environmental information may be directly measured by sensors provided in these electronic devices.

The environmental information is, for example, the following information.
- Weather, temperature, humidity, and electromagnetic wave strength
- Road surface shape information (acquired by the road surface shape information acquirer 1021)
- Road surface property information (acquired by the road surface property information acquirer 1022)
- Position information of the prosthetic leg 10

The road surface shape information is information representing the shape of a road surface of a walking route. The shape of the road surface is a concept including the gradient or height difference of the walking route and is roughly divided into a horizontal road surface without a gradient and an inclined road surface with a gradient. The horizontal road surface and the inclined road surface are further subdivided according to the presence or absence of unevenness. The inclined road surface having unevenness is typically a staircase having each unevenness as a step.

The road surface property information is information representing a property or a state of the road surface of the walking route. The property of the road surface includes a constituent (asphalt, soil, sand, wood, metal, grass, and the like) of the road surface and its property (hardness, elasticity, coefficient of friction, etc.). The state of the road surface includes, for example, a dry state of the road surface. When the information concerning the dry state of the road surface is not available via the information communication network, the dry state can be estimated based on available information such as weather, temperature, and humidity. For example, in a case where it has been raining until just before, and the temperature is not raised and the humidity is high while it is cloudy at present, it can be estimated that the road surface is wet. Note that the nature of the road surface can vary according to the state of the road surface. For example, in a state where the road surface is wet, the friction coefficient of the road surface decreases. As explained above, the property of the road surface changes according to other environmental information such as weather, temperature, and humidity.

The position information of the prosthetic leg 10 is acquired using a satellite positioning system such as a global positioning system (GPS). In this case, a GPS sensor provided in a smartphone, a wearable device, or the like carried by the user of the prosthetic leg 10 can be used. Note that the position of the prosthetic leg 10 can be calculated by integrating the acceleration measured by the acceleration sensor 75 of the prosthetic leg 10, but since this is a relative displacement from a start point of the integration, it is preferable to use the GPS in a case where it is desired to acquire an absolute position on the earth.

Next, three prosthetic leg systems that can be realized by the configuration illustrated in FIG. 5 are explained in order. The first system is a power consumption calculation system. The second system is a state estimation system. The third system is a physical condition estimation system.

The power consumption calculation system is a system that calculates power consumption of the battery 55 of the prosthetic leg 10 and includes the driving information acquirer 101, the environmental information acquirer 102, the route information acquirer 103, the product identification information acquirer 104, the power consumption calculator 105, the recommended route presenter 106, and the output unit 112 among the components of the system controller 100. In the following explanation, a case where these components of the system controller 100 are realized in a smartphone carried by the user of the prosthetic leg 10 is taken as an example.

The route information acquirer 103 acquires route information of a plurality of routes from a start point to an end point where the user wearing the prosthetic leg 10 can walk. The user of the prosthetic leg 10 uses any map search service, sets a start point and an end point with an operation on a screen of the smartphone, and searches for a plurality of candidate routes connecting the two points.

The product identification information acquirer 104 acquires product identification information of the prosthetic leg 10. Here, it is preferable to use a model number assigned to each model of a product of the prosthetic leg 10 as the product identification information. In addition, serial numbers assigned to identify individual products may be used. Since a mode of power consumption of the battery 55 of the prosthetic leg 10 differs depending on the configuration, that is, the model of the prosthetic leg 10, the calculation accuracy of power consumption for each product can be enhanced by using the product identification information.

For each candidate route acquired by the route information acquirer 103, the power consumption calculator 105 calculates, based on the road surface shape information acquired by the road surface shape information acquirer 1021 and the road surface property information acquired by the road surface property information acquirer 1022, power consumption of the battery 55 at the time when the user wearing the prosthetic leg 10 indicated by the product identification information walks on the route.

Most of the power consumption of the battery 55 of the prosthetic leg 10 relates to the driving of the cylinder 30 by the driving mechanism 40. Specifically, most electric power is consumed for opening and closing driving of each of the hydraulic valves 43 and 45 of the driving mechanism 40. Here, a case where the user wearing the prosthetic leg 10 walks on a horizontal road surface without a slope and a case where the user walks on an inclined road surface with a slope are compared. When the user walks on the horizontal road surface, the bending angle of the knee unit 22 for each step is small, and a load applied to the piston rod 34 of the cylinder 30 is also small. Therefore, since each of the hydraulic valves 43 and 45 is driven with a small opening/closing amount and a low load, power consumption is small. Conversely, when the user walks on the inclined road surface, the bending angle of the knee unit 22 for each step is large, and the load applied to the piston rod 34 of the cylinder 30 is also large. Therefore, since each of the hydraulic valves 43 and 45 is driven with a large opening and closing amount and a high load, power consumption is large. As explained above, the power consumption of the battery 55 of the prosthetic leg 10 greatly changes according to the shape of the road surface.

Therefore, the power consumption calculator 105 uses the road surface shape information when calculating power consumption of each candidate route. For example, the road surface shape is classified into a plurality of types, and a calculation formula of power consumption is set for each type. The type can be optionally set, but examples thereof include a horizontal road surface having no gradient, an inclined road surface having a gradient and no step, and a staircase road surface having a gradient and a step. Concerning the horizontal road surface, types subdivided according to a degree of unevenness the horizontal road surface may be provided. Concerning the inclined road surface, types subdivided according to the inclination angle of the inclined road surface may be provided. Concerning the staircase road surface, types subdivided according to the inclination angle and the height difference of a step of the staircase road surface may be provided. The calculation formula for the power consumption set for each type gives power consumption per unit distance at the time when the user walks on the road surface of the type. Note that, in a candidate route passing the inside of a building, when structural information of the inside of the building can be obtained, power consumption calculated based on the structural information may be used.

Here, the power consumption per unit distance of the horizontal road surface is represented as Ea, the power consumption per unit distance of the inclined road surface is represented as Eb, and the power consumption per unit distance of the staircase road surface is represented as Ec. When a road surface of a first candidate route is a horizontal road surface and a distance is D1, the power consumption is calculated by Ea × D1. When a road surface of a second candidate route is a staircase road surface and a distance is D2, the power consumption is calculated by Ec × D2.

In such power consumption calculation, each candidate route may be divided into small routes, and the power consumption may be calculated for each small route. For example, when a third candidate route is divided into three small routes of an inclined road surface with a distance D31, a horizontal road surface with a distance D32, and a staircase road surface with a distance D33, the power consumption is Eb × D31 + Ea × D32 + Ec × D33. That is, the power consumption of the candidate route is a sum of the power consumptions of the small routes.

As explained above, the road surface shape information is the most important in the calculation of the power consumption, but in addition to this, the calculation accuracy can be improved by considering the road surface property information and the product identification information. In a case where the dry state of the road surface is considered as the road surface property information, for example, in a case where the road surface is wet, the user of the prosthetic leg 10 walks with a larger load than usual so as not to slip or take his/her foot on the muddy ground during walking. Therefore, the load of the prosthetic leg 10 increases and the power consumption increases. In order to take such an influence into the calculation of the power consumption, for example, a correction coefficient α according to the dry state of the road surface is set. In a case where the road surface is completely dry, α is set to 1. In a case where the road surface is wet, α is set to be larger than 1. In the above example, when the power consumption calculated without considering the dry state of the road surface is Eb × D31 + Ea × D32 + Ec × D33, the power consumption calculated considering the dry state of the road surface is α × (Eb × D31 + Ea × D32 + Ec × D33). At this time, a different correction coefficient α may be set for each type of the road surface shape. When the correction coefficient of the horizontal road surface is represented as αa, the correction coefficient of the inclined road surface is represented as αb, and the correction coefficient of the staircase road surface is represented as αc, the power consumption is αb × Eb × D31 + αa × Ea × D32 + αc × Ec × D33. When the product identification information is considered in the calculation of the power consumption, the power consumptions Ea, Eb, and Ec of the road surface shape types may be set for each model of the product.

The recommended route presenter 106 presents a recommended route out of a plurality of candidate routes on the screen of the smartphone, based on the power consumption calculated by the power consumption calculator 105. Although the recommendation criterion can be optionally set, a route having the lowest power consumption, for example, is recommended out of a plurality of candidate routes. In addition, the power consumption of each candidate route may be compared with the amount of the remaining power at present of the battery 55 and, then, a candidate route with the shortest walking distance or required time may be recommended among candidate routes with less power consumptions than the amount of the remaining power.

The output unit 112 outputs the power consumption calculated by the power consumption calculator 105. For example, the power consumption is displayed on the screen of the smartphone, or the power consumption is transmitted to another communication device via the information communication network. The notifier 1121 makes a notification when the power consumption calculated by the power consumption calculator 105 exceeds the amount of the remaining power of the battery 55. This notification may be performed by highlighting a candidate route with power consumption exceeding the amount of the remaining power when the recommended route presenter 106 displays another candidate route together with the recommended route. In addition, this notification may be performed not only at the time of searching for a candidate route but also during actual walking on one of the routes. In this case, the power consumption calculator 105 calculates, in real time, required power to an end point of a route on which the user of the prosthetic leg 10 is actually walking and compares the calculated required power with the amount of the remaining power of the battery 55. Then, the notification is made when the required power to the end point exceeds the amount of the remaining power of the battery 55. This notification may be made not only to the user of the prosthetic leg 10 but also to an outsider in a remote place. For example, it is possible to notify a family or a prosthetist in a remote place that the user of the prosthetic leg 10 is in a situation requiring support. Note that when notifying the user, the notifier 1121 can also use the vibrator 85 provided in the prosthetic leg 10.

Subsequently, a state estimation system that is a second system that can be realized by the configuration illustrated in FIG. 5 is explained. The state estimation system is a system that estimates a state of the prosthetic leg 10 and includes the driving information acquirer 101, the environmental information acquirer 102, the estimator 107, and the output unit 112 among the components of the system controller 100. Here, the "state" of the prosthetic leg 10 is a concept including abnormality of the prosthetic leg 10 itself and danger of the user wearing the prosthetic leg 10.

The estimator 107 estimates the state of the prosthetic leg 10 based on driving information acquired by the driving information acquirer 101 and environmental information acquired by the environmental information acquirer 102. The output unit 112 outputs a result of the estimation of the estimator 107.

There are the following four types of the estimation in the estimator 107. A first estimation type is a case where no abnormality is recognized in both the driving information and the environmental information. At this time, the estimator 107 estimates that there is no abnormality in the prosthetic leg 10 and there is no danger for the user. A second estimation type is a case where abnormality is recognized in the driving information and no abnormality is recognized in the environmental information. At this time, the estimator 107 estimates that there is abnormality in the prosthetic leg 10 and estimates that there is danger for the user accordingly. The case where the abnormality is recognized in the driving information is, for example, a case where the prosthetic leg 10 is not normally operating and values of measurement information of the sensor group and internal information of the cylinder 30 or the driving mechanism 40 deviate from a predetermined normal range. At this time, the notifier 1121 of the output unit 112 immediately notifies the user of the prosthetic leg 10 that the prosthetic leg 10 is not normally operating and also notifies a family member or a prosthetist in a remote place according to necessity.

A third estimation type is a case where no abnormality is recognized in the driving information but abnormality is recognized in the environmental information. At this time, the estimator 107 estimates that there is no abnormality in the prosthetic leg 10 and estimates that there is danger for the user. In this type, since the value of the driving information is within a normal range, the prosthetic leg 10 is normally operating. However, since the abnormality is recognized in the environmental information, danger is involved in the user wearing the prosthetic leg 10 and walking under the environment.

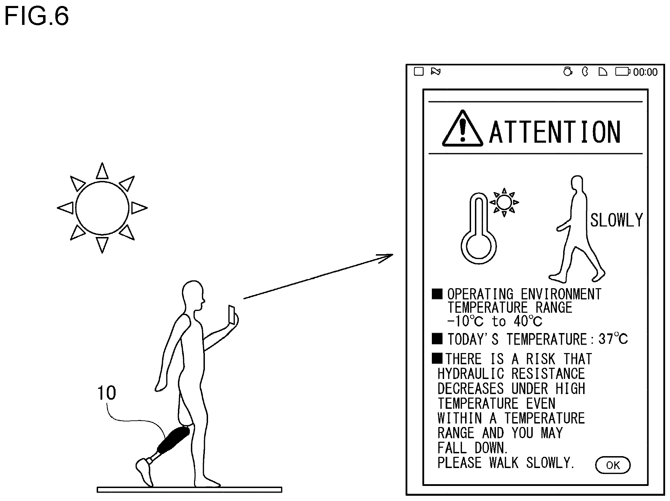

The case where the abnormality is recognized in the environmental information is, for example, a case where weather, temperature, humidity, and the intensity of an electromagnetic wave deviate from predetermined normal ranges. The case where the weather is abnormal includes a case where rain and wind are strong, a case where it is snowing, a case where sunlight or ultraviolet rays contained therein are strong, and the like. The case where the temperature and the humidity are abnormal is a case where the temperature and the humidity are higher than predetermined upper limit values and lower than predetermined lower limit values. The case where the intensity of the electromagnetic wave is abnormal is a case where the electromagnetic wave is stronger than a predetermined upper limit value. When such abnormality is recognized, the driving of the prosthetic leg 10 or the walking of the user wearing the prosthetic leg 10 may be adversely affected, so that the user needs to walk more carefully than at a normal time. Therefore, notifier 1121 of output unit 112 notifies the abnormality of the environmental information to the user to call the user's attention. FIG. 6 illustrates an example of such attention calling. Since temperature has risen while the user wearing the prosthetic leg 10 is walking on a flat road surface, the attention is called on the screen of the smartphone of the user. As shown on the screen, an operating environment temperature range of the prosthetic leg 10 is -10°C to 40°C, and the temperature of 37°C at present is within the range. However, since a temperature lower than 40°C (for example, 35°C) is set as an upper limit value of the temperature in the estimator 107, preliminary attention can be called before the temperature rises to 40°C. In addition, as shown in the text calling for attention, since hydraulic resistance decreases at high temperatures, the user is recommended to walk at a low speed for safety.

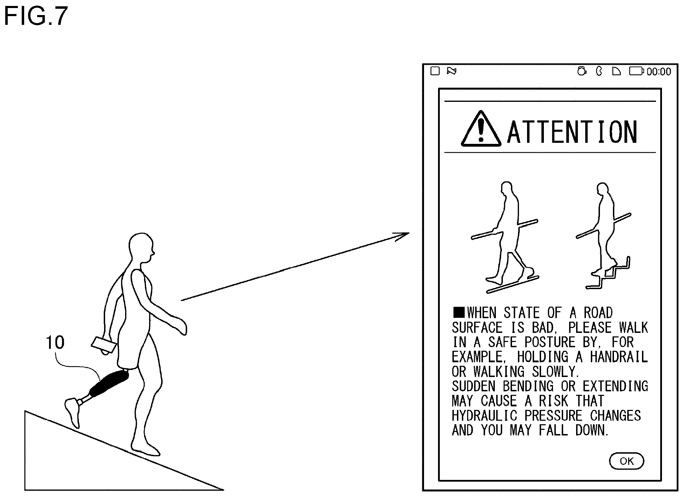

As another example in which the abnormality is recognized in the environmental information, even if neither of the environmental information regarding the weather or the like nor the environmental information regarding the situation of the road surface is recognized as being abnormal by itself, there is a case where user's attention should be called when the environmental information and the environmental information are combined. FIG. 7 illustrates an example of such attention calling. When recognizing that the user wearing the prosthetic leg 10 is walking on a loose slope from road surface shape information which is one of the environmental information and further recognizing the possibility of weak rainfall from information such as weather which is one of the environmental information, the estimator 107 estimates danger involved in the user wearing the prosthetic leg 10 and walking on a slope wet with rain, and the notifier 1121 calls attention on the screen of the smartphone of the user.

In the estimation of abnormality of the environmental information, walking information which is one of the driving information may be taken into consideration. For example, the estimator 107 considers a stride or walking speed indicated by the walking information and determines whether walking of the user at the stride or the walking speed is dangerous in light of the environmental information. In the example of FIG. 7, when the user walks at a small stride and low speed, there is little danger even if the slope is somewhat wet. Therefore, as a result of considering the stride or the walking speed, the estimator 107 may estimate that there is no danger. Conversely, in a case where the user is walking at a large stride or high speed, it is very dangerous if a slope gets wet. Therefore, the estimator 107 estimates that there is danger, and the notifier 1121 notifies the user. Note that, in this example, the estimator 107 is equivalent to storing a threshold of the driving information corresponding to the environmental information and performing estimation based on comparison of the threshold and the driving information. That is, the environmental information in this example is "being a gentle slope" (road surface shape information) and "having a possibility of weak rainfall" (weather information), and thresholds of the driving information "stride" and "walking speed" corresponding thereto are provided. In a case where the actual driving information "stride" and "walking speed" is larger than the thresholds, the estimator 107 estimates danger of the user.

A fourth estimation type is a case where abnormality is recognized in both of the driving information and the environmental information. At this time, the estimator 107 estimates that abnormality is present in the prosthetic leg 10 and the user has danger. Since this type has an extremely high risk, the notifier 1121 of the output unit 112 immediately notifies the user of the prosthetic leg 10 and stops walking. At the same time, the notifier 1121 may notify a family or a prosthetist in a remote place.

Next, a special example of state estimation by the state estimation system is explained.

In the first example, the estimator 107 performs estimation based on comparison of the driving information at present acquired by the driving information acquirer 101 and driving information in the past acquired by the driving information acquirer 101 within a predetermined range from the position indicated by the position information at present acquired by the environmental information acquirer 102. Here, the driving information compared between the present and the past is, for example, measurement information of the various sensors included in the sensor group. Since these pieces of measurement information have been obtained in the same environment in the present and the past, there is a possibility of abnormality or deterioration of the sensors in a case where there is deviation between the present and the past In this way, it is possible to effectively specify abnormality and deterioration of the sensors.

In the second example, the driving information acquirer 101 acquires, as the driving information, the temperature around the control board of the controller 50 that controls the driving mechanism 40, the environmental information acquirer 102 acquires the temperature around the prosthetic leg 10 as the environmental information, and the estimator 107 estimates the state of the control board based on the comparison of the temperature around the control board and the temperature around the prosthetic leg 10. When the control board has an abnormally high temperature compared to the outside air temperature, there is a high possibility that there is abnormality in the control board, and according to this example, the abnormality in the control board can be effectively specified.

In the third example, the driving information acquirer 101 acquires, as the driving information, a convergence time until the opening degrees of the hydraulic valves 43 and 45 converge to command values of the opening degrees, and the estimator 107 estimates a state of the prosthetic leg 10 based on the convergence time and the environmental information. The convergence time acquired as the driving information in this example becomes longer according to deterioration of the hydraulic valves 43 and 45. A long convergence time is not a big problem when walking speed is low. Therefore, when the environmental information (road surface shape information) indicates a road surface on which the user walks at low speed such as a slope, the estimator 107 does not estimate danger of the user. On the other hand, when the environmental information (road surface shape information) indicates a road surface on which the user can walk at high speed such as a horizontal road surface, the estimator 107 estimates danger of the user, and the notifier 1121 calls the user' attention to walk at low speed.

Next, a physical condition estimation system that is a third system that can be realized by the configuration illustrated in FIG. 5 is explained. The physical condition estimation system is a system that estimates a physical condition of the user of the prosthetic leg 10 and includes the driving information acquirer 101, the environmental information acquirer 102, the estimator 107, the reference information acquirer 108, the biological information acquirer 109, the determiner 110, the control information adjuster 111, and the output unit 112 among the components of the system controller 100.

The reference information acquirer 108 acquires reference information in which driving information, environmental information, and physical condition information of the user at the time when the user wore the same prosthetic leg 10 in the past are associated with each other. The reference information is unitarily recorded in the system memory 200 together with time information in the past when the reference information is recorded. The physical condition information of the user included in the reference information includes various kinds of biological information such as a body temperature, a heart rate, a blood pressure, a blood glucose level, a blood oxygen concentration, a respiration amount, a respiration frequency, a perspiration amount, a muscle potential, and a posture, and self-report information of the user regarding whether the physical condition is good or bad. The reference information records a correlation between the physical condition information and driving information of the prosthetic leg 10 and environmental information around the prosthetic leg 10 at that time.

The estimator 107 estimates the physical condition of the user at present based on comparison of the driving information and the environmental information at present while the user wears the prosthetic leg 10 with the reference information.

Specifically, first, reference information including environmental information similar to the environmental information at present is acquired. Here, a similarity criterion of the environmental information can be optionally set, but for example, several kinds of environmental information to be used for similarity determination are selected, and the similarity determination is performed based on a deviation degree (difference) of the those kinds of environmental information. As an example, when temperature, the intensity of an electromagnetic wave, and an inclination angle of a road surface are selected, differences between the past (at the time of recording the reference information) and the present are calculated, and a sum the differences is calculated. Then, in a case where the sum is less than a predetermined value, it is determined that the environmental information is similar. In the above calculation, weighting based on importance degrees may be performed on each environmental information.

After the reference information similar to the environmental information at present is acquired as explained above, the similarity determination of the driving information in the past and the driving information at present included in the reference information is performed. Similar to the above environmental information, a criterion for similarity determination can be optionally set. For example, as the driving information used for the similarity determination, a part or all of an average value per step of measurement information of various sensors, an average value per step of hydraulic resistance of each unit of the cylinder 30 or the driving mechanism 40, a stride, walking speed, a kicking force per step, an impact at landing, and an amount of remaining power of the battery 55 are selected.

In the two-stage similarity judgement explained above, since the reference information including the environmental information similar to the environmental information at present is extracted in the first-stage environmental information comparison step, the driving information usually has a similar result even in the second-stage driving information comparison step. However, when there is abnormality in the physical condition of the user of the prosthetic leg 10, the influence of the abnormality also appears in the driving information, and, as a result, the driving information is not similar in the second-stage comparison step. Therefore, through such a similarity determination step, the estimator 107 can detect abnormality in the physical condition of the user of the prosthetic leg 10.

To give several specific examples, in a case where the user walks in a similar environment but a step is smaller than usual, walking speed is low, a kicking force per step is weak, an impact at the time of landing is weak, or the like, there is a possibility that the physical condition of the user of the prosthetic leg 10 is bad. In addition, even in a case where various parameters of the prosthetic leg 10 such as an average value per step of the measurement information of the various sensors and an average value per step of the hydraulic resistance of each part of the cylinder 30 or the driving mechanism 40 deviate from the normal time despite walking in a similar environment, a change in physical condition from the time of recording the reference information is estimated. However, in this case, the physical condition may be better than usual. In addition, in a case where various parameters of the prosthetic leg 10 deviate from the normal time, there is a possibility of abnormality of the prosthetic leg 10 itself. However, by using the above state estimation system together, it is possible to identify abnormality of the prosthetic leg 10 itself and abnormality of the physical condition of the user of the prosthetic leg 10.

For the physical condition estimation by the estimator 107, the biological information, at present, of the user wearing the prosthetic leg 10 acquired by the biological information acquirer 109 can also be used. The biological information acquirer 109 acquires various kinds of biological information such as a body temperature, a heart rate, a blood pressure, a blood glucose level, a blood oxygen concentration, a respiratory amount, a respiratory frequency, a perspiration amount, a myoelectric potential, and a posture from various biological sensors provided in the prosthetic leg 10 or a smartphone or a wearable device used by the user of the prosthetic leg 10. The estimator 107 can accurately estimate physical condition of the user of the prosthetic leg 10 by referring to the biometric information at present.

The output unit 112 notifies an estimation result of the estimator 107. The user of the prosthetic leg 10 may be notified of the estimation result, but since it is considered that the user is aware of the physical condition of the user, it is important to notify the abnormality of the physical condition of the user to an outside different from the user, for example, a family or a prosthetist. Here, a criterion of notification is set so that the frequency of notification to the outsider does not become too high. Specifically, when the amount of movement of the user during a predetermined time is less than a predetermined amount, the determiner 110 determines to cause the output unit 112 to notify the result of the estimation by the estimator 107. That is, when the physical condition of the user is bad, it is considered that the movement amount decreases, and thus only when the movement amount is less than the predetermined amount, the estimation result of poor physical condition is notified to the outsider.

The control information adjuster 111 adjusts the control information of the prosthetic leg 10 according to the estimation result of the estimator 107. In particular, when the estimator 107 estimates that the physical condition of the user is bad, the control information is adjusted so that the user can easily walk. For example, it is conceivable to decrease a lock angle of the prosthetic leg 10 in order to facilitate walking at a small stride, to decrease hydraulic resistance by increasing the opening degrees of the hydraulic valves 43 and 45 so that the knee unit 22 can bend and stretch at a small load, and to shorten a necessary resting time for applying safety lock so that the user can easily rest in a half-sitting posture. Note that the control information adjuster 111 may store a plurality of predetermined adjustment patterns and select one out of the plurality of adjustment patterns according to an estimation result of the estimator 107.

Note that the adjustment of the control information of the prosthetic leg 10 may require permission from an outsider such as a prosthetist. In such a case, when notifying the poor physical condition to the outside, the output unit 112 requests permission for adjustment of the control information. Then, the control information adjuster 111 executes the adjustment of the control information only when the permission is obtained from the outsider.

The present invention is explained above based on the embodiment. It is to be understood by those skilled in the art that the embodiments are examples, various modifications can be made to combinations of the respective components and the respective processing processes, and such modifications are also within the scope of the present invention.

In the embodiment, the prosthetic leg 10 is explained as an example of the prosthetic limb, but the present invention can also be used for other prostheses such as a prosthetic arm. In particular, among the three systems that can be realized by the configuration of FIG. 5, the state estimation system and the physical condition estimation system can be realized even if the prosthetic leg 10 is replaced with a prosthetic arm.

The present invention covers the below clauses.
Clause 1: A power consumption calculation system for a prosthetic leg comprising: a route information acquirer (103) structured to acquire route information indicating a route from a start point to an end point on which a user wearing a prosthetic leg (10) can walk, the prosthetic leg (10) including a driving mechanism (40) driven by a battery (55); a road surface shape information acquirer (1021) structured to acquire road surface shape information representing a shape of a road surface of a route; a power consumption calculator (105) structured to calculate, based on the road surface shape information, power consumption of the battery at a time when the user wearing the prosthetic leg walks on the route; and an output unit (112) structured to output the calculated power consumption.
Clause 2: The power consumption calculation system according to clause 1, further comprising a road surface property information acquirer (1022) structured to acquire road surface property information representing a property or a state of the road surface of the route, wherein the power consumption calculator calculates power consumption of the battery based on the road surface shape information and the road surface property information.
Clause 3: The power consumption calculation system according to clause 1 or 2, further comprising a notifier (1121) structured to make a notification when the power consumption calculated by the power consumption calculator exceeds an amount of remaining power of the battery.
Clause 4: The power consumption calculation system according to any one of clauses 1 to 3, wherein the route information acquirer acquires route information of a plurality of routes, the road surface shape information acquirer acquires road surface shape information of the plurality of routes, the power consumption calculator calculates power consumption of the battery for each of the plurality of routes, and the power consumption calculation system further comprises a recommended route presenter (106) structured to present a recommended route out of the plurality of routes, based on the power consumption calculated by the power consumption calculator.
Clause 5: A state estimation system for a prosthetic limb (10) including a driving mechanism (40) that drives a joint (20), the state estimation system comprising: a driving information acquirer (101) structured to acquire driving information representing a driving state at present of the driving mechanism; an environmental information acquirer (102) structured to acquire environmental information, at present, around the prosthetic limb; an estimator (107) structured to estimate a state of the prosthetic limb based on the driving information and the environmental information; and an output unit (112) structured to output a result of the estimation.
Clause 6: The state estimation system according to clause 5, wherein the prosthetic limb is a prosthetic leg (10) including a knee joint (20) as the joint, the driving mechanism includes a sensor (70, 75) structured to measure at least one of acceleration during walking of a user wearing the prosthetic leg and a load applied to the prosthetic leg during the walking, the driving information acquirer acquires measurement information of the sensor as the driving information, and the estimator calculates a stride or walking speed of the user wearing the prosthetic leg based on the driving information, and estimates a state of the prosthetic leg in light of the environmental information.
Clause 7: The state estimation system according to clause 5 or 6, wherein the prosthetic limb is a prosthetic leg (10) including a knee joint (20) as the joint, and the environmental information acquirer acquires position information of the prosthetic leg at present as the environmental information, and the estimator estimates the state of the prosthetic leg based on comparison of driving information at present acquired by the driving information acquirer with driving information in past acquired by the driving information acquirer within a predetermined range from a position indicated by the position information at present.
Clause 8: The state estimation system according to any one of clauses 5 to 7, wherein the driving information acquirer acquires, as the driving information, temperature near a control board that controls the driving mechanism, the environmental information acquirer acquires temperature around the prosthetic limb as the environmental information, and the estimator estimates a state of the control board based on comparison of temperature near the control board with temperature around the prosthetic limb.
Clause 9: The state estimation system according to any one of clauses 5 to 8, wherein the prosthetic limb is a prosthetic leg (10) including a knee joint (20) as the joint and a hydraulic driving mechanism (40) including a hydraulic valve (43, 45) as the driving mechanism, and the driving information acquirer acquires, as the driving information, a convergence time until the opening degree of the hydraulic valve converges to the command value, and the estimator estimates the state of the prosthetic leg based on the convergence time and the environmental information.
Clause 10: The state estimation system according to clause 9, wherein the estimator stores a threshold of the driving information corresponding to the environmental information and estimates a state of the prosthetic leg based on comparison of the threshold with the driving information. Clause 11: The state estimation system according to clause 9 or 10, wherein the environmental information includes at least one of weather, temperature, humidity, intensity of electromagnetic waves, a shape of a road surface, a property of the road surface, and a state of the road surface.
Clause 12: A physical condition estimation system for estimating a physical condition of a user of a prosthetic limb (10) including a driving mechanism (40) that drives a joint (20), the physical condition estimation system comprising: a driving information acquirer (101) structured to acquire driving information representing a driving state of the driving mechanism; a reference information acquirer (108) structured to acquire reference information in which the driving information and physical condition information of the user are associated with each other when the user wore the prosthetic limb in the past; and an estimator (107) structured to estimate a physical condition of the user at present, based on comparison of the driving information at present while the user wears the prosthetic limb with the reference information.
Clause 13: The physical condition estimation system according to clause 12, further comprising an environmental information acquirer (102) structured to acquire environmental information around the prosthetic limb, wherein the reference information acquirer acquires reference information in which the environmental information at a time when the user wore the prosthetic limb in past is correlated with the driving information and the physical condition information of the user, and the estimator estimates a physical condition of the user at present, based on comparison of the driving information and the environmental information at present when the user wears the prosthetic limb and the reference information.
Clause 14: The physical condition estimation system according to clause 12 or 13, further comprising a biological information acquirer (109) structured to acquire biological information of the user wearing the prosthetic limb, wherein the estimator further estimates a physical condition of the user at present, based on the biological information at present.
Clause 15: The physical condition estimation system according to any one of clauses 12 to 14, further comprising an output unit (112) structured to output a result of the estimation. Clause 16: The physical condition estimation system according to clause 15, wherein the prosthetic limb is a prosthetic leg (10) including a knee joint (20) as the joint, and the physical condition estimation system further comprises a determiner (110) structured to determine to cause the output unit to output the estimation result when a movement amount of the user during a predetermined time is less than a predetermined amount.
Clause 17: The physical condition estimation system according to clause 15 or 16, wherein the output unit outputs the estimation result to an outsider different from the user. Clause 18: The physical condition estimation system according to any one of clauses 12 to 17, further comprising a control information adjuster (111) structured to adjust control information of the prosthetic limb according to a result of the estimation.
Clause 19: The physical condition estimation system according to clause 18, further comprising an output unit (112) structured to output a result of the estimation to an outsider different from the user, wherein the control information adjuster adjusts the control information when permission is obtained from the outsider.
Clause 20: The physical condition estimation system according to clause 18 or 19, wherein the control information adjuster stores a plurality of predetermined adjustment patterns and selects one of the plurality of adjustment patterns according to the estimation result.
Clause 21: The physical condition estimation system according to any one of clauses 12 to 20, wherein the prosthetic limb is a prosthetic leg (10) including a knee joint (20) as the joint and a hydraulic driving mechanism (40) including a hydraulic valve (43, 45) as the driving mechanism, and the driving information includes at least one of measurement information of a sensor (70, 75) that measures at least one of acceleration during walking of a user wearing the prosthetic limb and a load applied to the prosthetic leg during the walking, internal state information representing an internal state of the hydraulic driving mechanism, and walking information representing a walking state of the user estimated based on these pieces of information.

The functional configuration of each device explained in the embodiment can be realized by hardware resources or software resources, or by cooperation of hardware resources and software resources. A processor, a ROM, a RAM, and other LSIs can be used as the hardware resources. Programs such as an operating system and an application can be used as the software resources.

Among the embodiments disclosed in the present specification, those in which a plurality of functions is provided in a distributed manner may be provided by aggregating some or all of the plurality of functions, and conversely, those in which a plurality of functions is provided in an aggregated manner can be provided so that some or all of the plurality of functions are distributed. The present invention only has to be configured to achieve the object of the invention irrespective of whether the functions are aggregated or distributed.

## Claims

1. A state estimation system for a prosthetic limb (10) including a driving mechanism (40) that drives a joint (20), the state estimation system comprising:
a driving information acquirer (101) structured to acquire driving information representing a driving state at present of the driving mechanism;
an environmental information acquirer (102) structured to acquire environmental information, at present, around the prosthetic limb;
an estimator (107) structured to estimate a state of the prosthetic limb based on the driving information and the environmental information; and
an output unit (112) structured to output a result of the estimation.

2. The state estimation system according to claim 1, wherein
the prosthetic limb is a prosthetic leg (10) including a knee joint (20) as the joint,
the driving mechanism includes a sensor (70, 75) structured to measure at least one of acceleration during walking of a user wearing the prosthetic leg and a load applied to the prosthetic leg during the walking,
the driving information acquirer acquires measurement information of the sensor as the driving information, and
the estimator calculates a stride or walking speed of the user wearing the prosthetic leg based on the driving information, and estimates a state of the prosthetic leg in light of the environmental information.

3. The state estimation system according to claim 1 or 2, wherein
the prosthetic limb is a prosthetic leg (10) including a knee joint (20) as the joint, and
the environmental information acquirer acquires position information of the prosthetic leg at present as the environmental information, and
the estimator estimates the state of the prosthetic leg based on comparison of driving information at present acquired by the driving information acquirer with driving information in past acquired by the driving information acquirer within a predetermined range from a position indicated by the position information at present.

4. The state estimation system according to any one of claims 1 to 3, wherein
the driving information acquirer acquires, as the driving information, temperature near a control board that controls the driving mechanism,
the environmental information acquirer acquires temperature around the prosthetic limb as the environmental information, and
the estimator estimates a state of the control board based on comparison of temperature near the control board with temperature around the prosthetic limb.

5. The state estimation system according to any one of claims 1 to 4, wherein
the prosthetic limb is a prosthetic leg (10) including a knee joint (20) as the joint and a hydraulic driving mechanism (40) including a hydraulic valve (43, 45) as the driving mechanism, and
the driving information acquirer acquires, as the driving information, a convergence time until the opening degree of the hydraulic valve converges to the command value, and
the estimator estimates the state of the prosthetic leg based on the convergence time and the environmental information.

6. The state estimation system according to claim 5, wherein
the estimator stores a threshold of the driving information corresponding to the environmental information and estimates a state of the prosthetic leg based on comparison of the threshold with the driving information.

7. The state estimation system according to claim 5 or 6, wherein the environmental information includes at least one of weather, temperature, humidity, intensity of electromagnetic waves, a shape of a road surface, a property of the road surface, and a state of the road surface.
